# EUROPEAN PATENT SPECIFICATION

(11) **EP 2 066 301 B1**
(45) Date of publication and mention of the grant of the patent: **25.08.2010**
(21) Application number: 07820290.0
(22) Date of filing: 18.09.2007
(51) Int. Cl.: A61K 9/00, A61K 47/18, A61K 31/16

(54) **INJECTABLE PHARMACEUTICAL NIMESULIDE SOLUTIONS**
INJIZIERBARE PHARMAZEUTISCHE NIMESULID-LÖSUNGEN
SOLUTIONS PHARMACEUTIQUES INJECTABLES DE NIMÉSULIDE

(30) Priority: 22.09.2006 EP 06121134
(43) Date of publication of application: 10.06.2009
(73) Proprietor: Pharmatex Italia Srl, 20121 Milano (IT)
(72) Inventor: DE TOMMASO, Vincenzo, I-20052 Monza (IT)
(74) Representative: Serravalle, Marco
(86) International application number: PCT/EP2007/059827
(87) International publication number: WO 2008/034819

(56) References cited:
- WO-A-91/17774
- WO-A-95/34533
- WO-A-99/41233
- WO-A-2005/116086
- US-A1- 2005 020 688
- DATABASE CA CHEMICAL ABSTRACTS SERVICE, COLUMBUS, OHIO, US; XP002441656 retrieved from STN Database accession no. 143: 292603 & RU 2 260 432 A (OK. AKTS. OBSSHCHT. "NIZHEGORODSKII KHIMIKO-FARMAT. ZAV.") 20 September 2005 (2005-09-20)

## Description

The present invention relates to a nimesulide solution, suitable for injections, comprising nimesulide and an aminosugar.

Nimesulide, whose chemical name is 4-nitro-2-phenoxymethanesulfonanilide, is a well known non-steroidal anti-inflamatory and antipyretic dug, useful in the presence of inflammation and pain. However, it is well known that nimesulide is sparingly soluble in water and this fact makes it more difficult oral or parenteral administration of nimesulide. Furthermore, the low solubility in water negatively influences its bioavailability.

WO 91/17774 and WO 94/02177 describe inclusion compounds of nimesulide with cyclodextrins. By using such inclusion compounds, solubility of nimesulide is increased to 0.61 mg/ml, which is about 20 times higher than pure crystalline nimesulide.

WO 95/34533 discloses aqueous solutions of nimesulide and L-lysine. The solubility of this salt is 5.42 mg/ml, and further increases to 30.16 mg/ml in the presence of gamma-cyclodextrin at a concentration of 200 mM.

WO 99/41233 discloses a preparation of nimesulide-aminosugar adducts which can be used in the preparation of injectable solutions. The adduct after preparation must be dried because nimesulide is not stable in solution at high pH values. Furthermore, the use of a solution having pH around 10 for injections, would result in considerable pain in the injection area.

The composition according to the present invention allows the achievement of a nimesulide solution at concentration higher than 100 mg/ml. These formulations are obtained through the following steps:
1) Mixing a non ionic surfactant with an organic co-solvent.
2) Adding nimesulide to the mixture obtained in 1) and leaving under stirring for 15'.
3) Preparing a solution of aminosugar in water.
4) Adding the solution prepared in 3) to the suspension of nimesulide prepared in 2), while measuring the pH.

At a pH between 7.5 and 9.5 nimesulide is completely dissolved. Preferably, in step 4) the pH is maintained between 6 and 9.5, most preferably between 8.0 and 9.0, by adding, if necessary, a buffer to the solution. The solution is brought to the final volume with water, is then filtered through a 0.22 µm sterilizing membrane and the solution is placed in 1 ml ampules.

The non-ionic surfactant used in step 1) can be any non-ionic surfactant suitable for pharmaceutical compositions. Examples of preferred non-ionic surfactants are glycofurol, polyethylenglycol 200, polyethylenglycol 300 and polyethylenglycol 400; the most preferred non-ionic surfactant is glycofurol.

The amount of non-ionic surfactant in the final solution is preferably from 1 to 50 % w/v, preferably from 10 to 45% w/v.

The organic co-solvent is a compound which is highly soluble in water and is a good solvent for nimesulide. Examples of preferred co-solvents are propylene glycol, butylene glycol, hexamethylene glycol, glycerol, N-dimethylacetamide; most preferred is propylene glycol.

The amount of co-solvent in the final composition preferably varies from 5 to 70% w/v, most preferably from 15 to 60% w/v.

The sum of the amount of non-ionic surfactant and organic co-solvent preferably ranges from 30% w/v to 95% w/v, preferably from 40% w/v to 90% w/v.

The pH of the composition is preferably comprised between 6 and 9.5, most preferably between 8.0 and 9.0.

Preferred examples of aminosugars useful in the present invention are: glucamine, glucosamine, chondrosamine and their N-alkyl derivatives such as N-methylglucamine.

The aminosugar used in the present invention is essential in the solubilization of nimesulide. The molar ratio between aminosugar and nimesulide preferably varies between 0.5:1 and 10:1. If the amount of aminosugar is too low, the solubility of nimesulide will be lower than desired. However, if the amount of amino sugar is too high, the pH will increase above 9.5 and it will be necessary to add a buffer to the solution such as a citrate or a phosphate or any other pharmaceutically acceptable buffer.

The nimesulide solution according to the invention can further comprise other ingredients. For example, it is possible to add cyclodextrins as known in the art.

The concentration of nimesulide in the solutions according to the invention is higher than 40 mg/ml, preferably higher than 70 mg/ml and most preferably higher than 80 mg/ml.

The solution according to the invention can also contain other ingredients. For example, it could be useful to add a local anaesthetic such as lidocaine, bupivacaine, mepivacaine, articaine, cinchocaine, etdocaine, levobupivacaine, oxetacaine, prilocaine, ropivacaine, tolycaine, trimecaine, amilocaine, proponacaine, proximetacaine, benzocaine, butacaine, butoxicaine, butylaminobenzoate, chloroprocaine, paretoxycaine, procaine, propoxicaine, tetracaine, tricaine.

The following example illustrates the invention.

### Example 1

### Preparation of an injectable nimesulide solution

1.65 kg of glycofurol and 2.150 kg of propylene glycol are placed under stirring in a vessel. 500 g of nimesulide are added and the suspension is left under stirring for 15'. In another vessel 600 ml of distilled water are placed and 370 g of N-methylglucamine are added thereto. The N-methylglucamine solution is slowly added to the nimesulide suspension, while measuring the pH.

During addition of the aminosugar, it is possible to note the solubilization of nimesulide. The solubilization is completed when reaching a pH of 8.5. After completion of the addition, the pH is comprised between 8.5 and 9.0. The solution is brought to a volume of 5 l by addition of distilled water. The solution is then filtered through a 0.22 µm sterilizing membrane and it is placed in 1 ml ampules.

Composition per 1 ml:

| | |
|---|---|
| Nimesulide | 100 mg |
| Glycofurol | 330 mg |
| Propylene glycol | 430 mg |
| N-methylglucamine | 74 mg |
| Water | up to 1ml volume |

## Claims

1. Injectable pharmaceutical nimesulide solution comprising: water, nimesulide, an aminosugar, an organic co-solvent and a non-ionic surfactant, wherein the concentration of nimesulide in the solution is higher than 40 mg/ml.

2. Solution according to claim 1 wherein the concentration of nimesulide in the solution is higher than 70 mg/ml.

3. Solution according to claims 1-2 wherein the amino sugar is selected form glucamine, glucosamine, chondrosamine and the N-alkylderivatives thereof.

4. Solution according to claims 1-3 wherein the molar ratio between aminosugar and nimesulide varies between 1:0.5 and 10:1.

5. Solution according to claims 1-4 wherein the organic co-solvent is propylene glycol.

6. Solution according to claims 1-5 wherein the non-ionic surfactant is glycofurol.

7. Solution according to claim 1-6 wherein the amount of co-solvent in the final composition vary from 5 to 70% w/v, preferably from 15 to 60% w/v

8. Solution according to claim 1-7 further comprising a local anaesthetic selected from: lidocaine, bupivacaine, mepivacaine, articaine, cinchocaine, etdocaine, levobupivacaine, oxetacaine, prilocaine, ropivacaine, tolycaine, trimecaine, amilocaine, proponacaine, proximetacaine, benzocaine, butacaine, butoxicaine, butylaminobenzoate, chloroprocaine, paretoxycaine, procaine, propoxicaine, tetracaine, tricaine or combinations thereof.

9. Solution according to claim 1 further comprising cyclodextrins.

10. Process for the preparation of nimesulide solutions, comprising the following steps:
a. Mixing a non ionic surfactant with an organic co-solvent.
b. Adding nimesulide to the mixture obtained in a) and leaving under stirring for 15 min.
c. Preparing a solution of aminosugar in water.
d. Adding the solution prepared in c) to the suspension of nimesulide prepared in b), while measuring the pH.

## Patentansprüche

1. Injizierbare pharmazeutische Nimesulidlösung, enthaltend Wasser, Nimesulid, einen Aminozucker, ein organisches Cosolvens und ein nichtionisches Tensid, worin die Nimesulid-Konzentration in der Lösung größer ist als 40 mg/ml.

2. Lösung gemäß Anspruch 1, worin die Nimesulid-Konzentration in der Lösung größer ist als 70 mg/ml.

3. Lösung gemäß Ansprüchen 1-2, worin der Aminozucker ausgewählt wird aus Glucamin, Glucosamin, Chondrosamin und den N-Alkylderivaten davon.

4. Lösung gemäß den Ansprüchen 1-3, worin das molare Verhältnis zwischen Aminozucker und Nimesulid zwischen 1:0,5 und 10:1 variiert.

5. Lösung gemäß Ansprüchen 1-4, worin das organische Cosolvens Propylenglykol ist.

6. Lösung gemäß Ansprüchen 1-5, worin das nichtionische Tensid Glycofurol ist.

7. Lösung gemäß Ansprüchen 1-6, worin die Cosolvens-Menge in der Endzusammensetzung von 5 bis zu 70 % (w/v), vorzugsweise von 15 bis zu 60 % (w/v) variiert.

8. Lösung gemäß Anspruch 1-7, weiterhin enthaltend ein lokales Anästhetikum ausgewählt aus: Lidocain, Bupivacain, Mepivacain, Articain, Cinchocain, Etidocain, Levobupivacain, Oxetacain, Prilocain, Ropivacain, Tolycain, Trimecain, Amylocain, Propanocain, Proxymetacain, Benzocain, Butacain, Butoxycain, Butylaminobenzoat, Chloroprocain, Paretoxycain, Procain, Propoxycain, Tetracain, Tricain oder Kombinationen davon.

9. Lösung gemäß Anspruch 1, weiterhin enthaltend Cyclodextrine.

10. Verfahren zur Herstellung von Nimesulid-Lösungen, enthaltend die folgenden Schritte:
a. Mischen eines nichtionischen Tensids mit einem organischen Cosolvens.
b. Zugabe von Nimesulid zu der in a) erhaltenen Mischung und für 15 min rühren lassen.
c. Vorbereitung einer Lösung von Aminozucker in Wasser.
d. Zugabe der in c) vorbereiteten Lösung zu der in b) vorbereiteten Nimesulid-Suspension, wobei der pH gemessen wird.

## Revendications

1. Solution pharmaceutiquement injectable de nimésulide comprenant : de l'eau, du nimésulide, un sucre aminé, un co-solvant organique et un agent tensio-actif non ionique, la concentration de nimésulide dans la solution étant supérieure à 40 mg/ml.

2. Solution selon la revendication 1, dans laquelle la concentration de nimésulide dans la solution est supérieure à 70 mg/ml.

3. Solution selon l'une des revendications 1 et 2, dans laquelle le sucre aminé est choisi parmi la glucamine, la glucosamine, la chondrosamine et leurs dérivés N-alkylés.

4. Solution selon l'une des revendications 1 à 3, dans laquelle le rapport molaire entre le sucre aminé et le nimésulide varie entre 1:0,5 et 10:1.

5. Solution selon l'une des revendications 1 à 4, dans laquelle le co-solvant organique est le propylène glycol.

6. Solution selon l'une des revendications 1 à 5, dans laquelle l'agent tensio-actif non ionique est le glycofurol.

7. Solution selon l'une des revendications 1 à 6, dans laquelle la quantité de co-solvant dans la composition finale varie de 5 à 70 % p/v, de préférence de 15 à 60 % p/v.

8. Solution selon l'une des revendications 1 à 7, comprenant en outre un anesthésique local choisi parmi : la lidocaïne, la bupivacaïne, la mépivacaïne, l'articaïne, la cinchocaïne, l'etdocaïne, la lévobupivacaïne, l'oxétacaïne, la prilocaïne, la ropivacaïne, la tolycaïne, la trimécaïne, l'amilocaïne, la proponacaïne, la proximétacaïne, la benzocaïne, la butacaïne, la butoxicaïne, le butylaminobenzoate, la chloroprocaïne, la parétoxycaïne, la procaïne, la propoxicaïne, la tétracaïne, la tricaïne ou leurs combinaisons.

9. Solution selon la revendication 1, comprenant en outre des cyclodextrines.

10. Procédé de préparation de solutions de nimésulide, comprenant les étapes suivantes consistant à :
a. Mélanger un agent tensio-actif non ionique avec un co-solvant organique ;
b. Ajouter du nimésulide au mélange obtenu en a) et laisser sous agitation pendant 15 minutes ;
c. Préparer une solution de sucre aminé dans de l'eau ;
d. Ajouter la solution préparée en c) à la suspension de nimésulide préparée en b), tout en mesurant le pH.
